# EUROPÄISCHE PATENTSCHRIFT

(11) **EP 2 300 060 B1**
(45) Veröffentlichungstag und Bekanntmachung des Hinweises auf die Patenterteilung: **04.09.2013**
(21) Anmeldenummer: 09780113.8
(22) Anmeldetag: 03.07.2009
(51) Int. Cl.: C08F 220/06, A61L 15/60, A61L 15/22, C08F 2/01, B01J 19/00, C07C 51/41

(54) **VERFAHREN ZUR HERSTELLUNG WASSERABSORBIERENDER POLYMERPARTIKEL**
METHOD FOR PRODUCING WATER-ABSORBING POLYMER PARTICLES
PROCÉDÉ DE FABRICATION DE PARTICULES POLYMÈRES ABSORBANT L'EAU

(30) Priorität: 11.07.2008 US 79991 P
(43) Veröffentlichungstag der Anmeldung: 30.03.2011
(73) Patentinhaber: BASF SE, 67056 Ludwigshafen (DE)
(72) Erfinder: WEISMANTEL, Matthias, 63637 Jossgrund (DE); FUNK, Rüdiger, 65527 Niedernhausen (DE); BLAIR, Leigh R., Greenwood Springs MS38848 (US); HEITZHAUS, Kevin D., Suffolk VA 23435 (US)
(86) Internationale Anmeldenummer: PCT/EP2009/058376
(87) Internationale Veröffentlichungsnummer: WO 2010/003884

(56) Entgegenhaltungen:
- WO-A-2007/028751
- DE-A1-102005 042 608
- DE-A1-102007 055 086

## Beschreibung

### Beschreibung

Die vorliegende Erfindung betrifft ein Verfahren zur Herstellung wasserabsorbierender Polymerpartikel, wobei das Monomer neutralisiert und mittels eines indirekten Wärmeaustauschers gekühlt wird.

Wasserabsorbierende Polymere werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet und bestehen aus hydrophilen Polymeren, die so stark vernetzt sind, dass sie nicht mehr löslich sind.

Die Herstellung der wasserabsorbierenden Polymere wird beispielsweise in der Monographie "Modern Superabsorbent Polymer Technology", F.L. Buchholz und A.T. Graham, Wiley-VCH, 1998, Seiten 71 bis 103, und in Ullmann's Encyclopedia of Industrial Chemistry, 6. Auflage, Band 35, Seiten 73 bis 93, beschrieben.

Sowohl bei der kontinuierlichen als auch bei der diskontinuierlichen Polymerisation wird üblicherweise teilneutralisierte Acrylsäure als Monomer verwendet. Geeignete Neutra-lisationsverfahren werden beispielsweise in EP 0 372 706 A2, EP 0 574 260 A1, WO 2003/051415 A1, EP 1 470 905 A1, WO 2007/028751 A1, WO 2007/028746 A1 und WO 2007/028747 A1 beschrieben.

EP 0 372 706 A2 beschreibt ein dreistufiges Neutralisationsverfahren, bei dem in einer ersten Stufe Acrylsäure und Natronlauge gleichzeitig dosiert werden, so dass ein Neutralisationsgrad von 75 bis 100 mol-% eingehalten wird, in einer zweiten Stufe der Neutralisationsgrad auf 100,1 bis 110 mol-% angehoben wird um in der eingesetzten Acrylsäure als Verunreinigung enthaltene Diacrylsäure zu hydrolysieren, und in einer dritten Stufe durch Zusatz von weiterer Acrylsäure ein Neutralisationsgrad von 20 bis 100 mol-% eingestellt wird.

EP 0 574 260 A1 offenbart auf Seite 7, Zeilen 38 bis 41, dass bei der Neutralisation vorteilhaft Natronlauge vorgelegt und anschließend Acrylsäure unter Kühlung zugesetzt wird.

WO 2003/051415 A1 lehrt ein Verfahren zur Herstellung wasserabsorbierender Polymere, bei dem die Monomerlösung eine Mindesttemperatur von 40°C aufweist.

EP 1 470 905 A1 beschreibt in den Beispielen die kontinuierliche Neutralisation von Acrylsäure unmittelbar vor dem Polymerisationsreaktor. Aufgrund der Neutralisationswärme steigt die Temperatur dabei auf 95°C.

WO 2007/028751 A1 offenbart ein Verfahren zur kontinuierlichen Neutralisation, wobei die bei der Neutralisation auftretenden Temperaturspitzen minimiert werden.

WO 2007/028746 A1 beschreibt ein kontinuierlich überwachtes Neutralisationsverfahren.

WO 2007/028747 A1 lehrt die Verwendung einer vorneutralisierten Monomerlösung zur Herstellung von Monomerlösungen mit unterschiedlichem Neutralisationsgrad.

Nachteilig bei der Verwendung von warmen bzw. heißen Monomerlösungen ist deren hohe Polymerisationsneigung und die damit verbundene Tendenz zur Bildung von Polymerablagerungen in den Anlagenteilen vor dem eigentlichen Polymerisationsreaktor. Daher werden vorzugsweise kalte Monomerlösungen eingesetzt. Aber auch bei Verwendung kalter Monomerlösungen, insbesondere bei Monomerlösungen mit hohen Feststoffgehalt, werden in den Anlagenteilen vor dem eigentlichen Polymerisationsreaktor nach längerem Betrieb unerwünschte Salzablagerungen gefunden.

Aufgabe der vorliegenden Erfindung war die Bereitstellung eines verbesserten, insbesondere betriebssicheren, Verfahrens zur Herstellung wasserabsorbierender Polymerpartikel.

Gelöst wurde die Aufgabe durch ein Verfahren zur Herstellung wasserabsorbierender Polymerpartikel durch Polymerisation einer Monomerlösung, enthaltend
a) mindestens ein ethylenisch ungesättigtes, säuregruppentragendes Monomer,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
wobei das Monomer a) zumindest teilweise neutralisiert wird und die Neutralisationswärme zumindest teilweise mittels eines indirekten Wärmeaustauschers mittels eines Kühlmediums abgeführt wird, dadurch gekennzeichnet, dass die spezifische Kühlleistung des Wärmeaustauschers weniger als 5 W/cm² beträgt.

Die spezifische Kühlleistung ist dabei der Quotient aus Gesamtkühlleistung des Wärmeaustauschers und Gesamtwärmeaustauscherfläche des Wärmeaustauschers.

Die spezifische Kühlleistung des Wärmeaustauschers beträgt besonders bevorzugt weniger als 2 W/cm², ganz besonders bevorzugt weniger als 1 *W*/*cm².* Die Temperatur des Kühlmediums, insbesondere am Eingang des Wärmeaustauschers, beträgt vorzugsweise mindestens 10°C, besonders bevorzugt mindestens 15°C, ganz besonders bevorzugt mindestens 20°C. Die Monomerlösung wird im Wärmeaustauscher vorzugsweise auf weniger als 40°C, besonders bevorzugt auf weniger als 35°C, ganz besonders bevorzugt auf weniger als 30°C, abgekühlt. Der Wärmeaustauscher wird vorzugsweise im Gegenstrom betrieben.

Der vorliegenden Erfindung liegt die Erkenntnis zugrunde, dass die Löslichkeitsgrenze im Wärmeaustauscher durch Abkühlung partiell überschritten werden kann, obwohl dies aufgrund der in der in der Neutralisation gemessenen Temperaturen noch nicht der Fall sein sollte. Dabei kristallisiert Salz des neutralisierten ethylenisch ungesättigten, säuregruppentragenden Monomeren. Insbesondere bei Monomerlösungen mit hohem Feststoffgehalt lösen sich diese Kristalle nur sehr langsam und führen zu unerwünschten Salzablagerungen in Rohrleitungen und Behältern.

Besonders vorteilhaft ist das erfindungsgemäße Verfahren daher bei einem Wassergehalt der Monomerlösung von vorzugsweise höchstens 70 Gew.-%, besonders bevorzugt höchstens 65 Gew.-%, ganz besonders bevorzugt höchstens 60 Gew.-%.

Besonders vorteilhaft ist das erfindungsgemäße Verfahren auch bei einem Neutralisationsgrad von mindestens 50 mol-%, besonders bevorzugt mindestens 60 mol-%, ganz besonders bevorzugt mindestens 65 mol-%.

Der Neutralisationsgrad ist das Molverhältnis von neutralisiertem ethylenisch ungesättigtem, säuregruppentragendem Monomer nach der Neutralisation zur Gesamtmenge eingesetztem ethylenisch ungesättigten, säuregruppentragendem Monomeren vor der Neutralisation multipliziert mit 100%.

Die Temperatur des der Neutralisation zugeführten ethylenisch ungesättigten, säuregruppentragenden Monomeren beträgt üblicherweise von 0 bis 40°C, vorzugsweise von 5 bis 35°C, besonders bevorzugt von 10 bis 30°C, ganz besonders bevorzugt von 15 bis 25°C, wobei auf einen ausreichenden Abstand zu Schmelzpunkt zu achten ist. Bei Verwendung von Acrylsäure sollte eine Temperatur von 15°C auf keinen Fall unterschritten werden.

Als Base ist wässriges Alkali bevorzugt. Wässriges Alkali sind alle alkalisch reagierenden wässrigen Lösungen, d.h. wässrige Lösungen mit einem pH-Wert von mindestens 8, vorzugsweise mindestens 10, besonders bevorzugt mindestens 12, ganz besonders bevorzugt mindestens 14.

Die im wässrigen Neutralisationsmittel einsetzbaren alkalischen Salze sind vorzugsweise Alkalimetallhydroxide, Alkalimetalloxide, Alkalimetallcarbonate und Alkalimetallhydrogencarbonate sowie deren Mischungen. Statt Alkalimetallsalzen können auch Ammoniumsalze verwendet werden. Natrium und Kalium als Alkalimetalle sind besonders bevorzugt, ganz besonders bevorzugt jedoch Natriumhydroxid, Natriumcarbonat oder Natriumhydrogencarbonat sowie deren Mischungen. Vorzugsweise werden wässrige Lösungen eingesetzt. Üblicherweise beträgt der Anteil des alkalischen Salzes in der wässrigen Lösung mindestens 10 Gew.-%, vorzugsweise mindestens 20 Gew.-%, bevorzugt mindestens 30 Gew.-%, ganz besonders bevorzugt mindestens 40 Gew.-%.

Die Temperatur des wässrigen Alkali beträgt üblicherweise von 0 bis 45°C, vorzugsweise von 5 bis 40°C, besonders bevorzugt von 10 bis 35°C, ganz besonders bevorzugt von 15 bis 30°C, wobei Übersättigungen und damit Ausfällungen zu vermeiden sind.

Beträgt der Alkaligehalt des wässrigen Alkali mindestens 25 Gew.-%, so sind höhere Temperaturen vorteilhaft, üblicherweise von 10 bis 60°C, vorzugsweise von 20 bis 55°C, besonders bevorzugt von 30 bis 50°C, ganz besonders bevorzugt von 40 bis 45°C.

Die Neutralisation wird vorzugsweise kontinuierlich durchgeführt. Dies bedeutet, dass dem Neutralisationsbereich ethylenisch ungesättigtes, säuregruppentragendes Monomer und/oder Base zugeführt wird und dem Neutralisationsbereich gleichzeitig neutralisierte Lösung entnommen wird. An- und Abfahrvorgänge des kontinuierlichen Neutralisationsverfahrens sind hiervon selbstverständlich ausgenommen.

Der Neutralisationsbereich ist der Bereich, in dem die Neutralisation weitgehend stattfindet, d.h. der Bereich in dem ethylenisch ungesättigtes, säuregruppentragendes Monomer und Base unter Salzbildung reagieren (Neutralisation).

Die Neutralisation ist weitgehend abgeschlossen, wenn der Umsatz der Neutralisation mindestens 90%, vorzugsweise mindestens 95%, bevorzugt mindestens 98%, ganz besonders bevorzugt mindestens 99%, beträgt.

Der Abstand zwischen Neutralisation und Polymerisation beträgt üblicherweise mindestens 1 m, vorzugsweise mindestens 5 m, besonders bevorzugt mindestens 10 m, ganz besonders bevorzugt mindestens 20 m, und üblicherweise nicht mehr als 100 m, wobei der Abstand die Länge der Strecke zwischen Alkalidosierung in der Neutralisation und dem Polymerisationsreaktor ist.

Zusätzlich kann die neutralisierte Lösung mit Wasser verdünnt werden. Über die Verdünnung mit Wasser kann der Feststoffgehalt der neutralisierten Lösung während oder nach der Neutralisation eingestellt werden.

Die Temperatur des Wassers beträgt üblicherweise von über 0 bis 40°C, vorzugsweise von 5 bis 35°C, besonders bevorzugt von 10 bis 30°C, ganz besonders bevorzugt von 15 bis 25°C.

Vorzugsweise werden Wasser und Base vorgemischt. In diesem Fall kann die dabei freiwerdende Lösungswärme bereits vor der Neutralisation, beispielsweise mittels geeigneter Wärmeaustauscher, abgeführt werden.

In einer besonders bevorzugten Ausführungsform der vorliegenden Erfindung wird ein Teil der neutralisierten Lösung in die Neutralisation rückgeführt.

Durch die Rückführung kann die Neutralisationswärme und die Lösungswärme besser verteilt und Temperaturspitzen (Peaktemperatur) in der Mischung niedrig gehalten werden. Der Anteil rückgeführter neutralisierter Lösung beträgt üblicherweise von 25 bis 99%, vorzugsweise von 33 bis 98%, besonders bevorzugt von 50 bis 95%, ganz besonders bevorzugt von 80 bis 90%, jeweils bezogen auf die neutralisierte Lösung.

Das ethylenisch ungesättigte, säuregruppentragende Monomer, die Base und optional das Wasser können an beliebigen Stellen in die rückgeführte neutralisierte Lösung dosiert werden. Vorzugsweise werden die Flüssigkeiten nacheinander dosiert, besonders bevorzugt Base und ethylenisch ungesättigtes, säuregruppentragendes Monomer nacheinander oder Wasser, Base und ethylenisch ungesättigtes, säuregruppentragendes Monomer nacheinander.

Vorteilhaft wird mindestens eines der Edukte über zwei oder mehr getrennte Zugabestellen dosiert.

Beispielsweise können die Edukte über zwei, drei, vier, fünf oder sechs Zugabestellen dosiert werden, wobei die Zugabestellen vorzugsweise so angeordnet sind, dass sie eine gemeinsame Achse aufweisen (für zwei Zugabestellen) oder einen symmetrischen Stern bilden (für mindestens drei Zugabestellen) und die Achse bzw. Stern senkrecht zur Flussrichtung der neutralisierten Lösung befindet (Mehrfachzugabestellen).

Besonders vorteilhaft wird die Base dosiert, wenn zwei, drei oder vier Mehrfachzugabestellen hintereinander angeordnet werden.

Das Aufteilen in mehrere Zugabestellen bewirkt eine gleichmäßigere Durchmischung und niedrigere Temperaturspitzen, was die Gefahr unerwünschter Polymerisation vermindert.

In einer weiteren Ausführungsform werden Wasser und Base so dosiert, dass das Wasser die Base beim Eintritt in die Neutralisation umhüllt. Dazu können beispielsweise zwei ineinander gesteckte Rohre verwendet werden, wobei die Base über das Innenrohr und das Wasser über den Ringspalt zwischen innerem und äußeren Rohr dosiert werden.

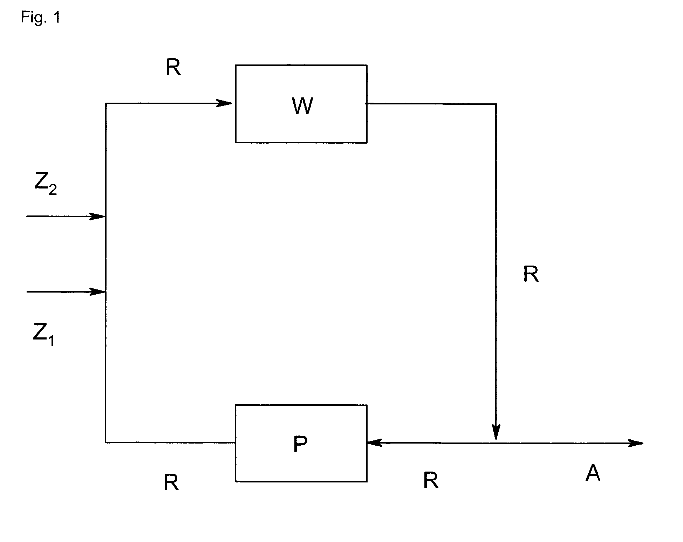

Eine beispielhafte erfindungsgemäße Neutralisation zeigt Figur 1, wobei die Bezugszeichen folgende Bedeutungen haben:
- Z₁ bis Z₂: Zuführungen für Edukte 1 und 2
- A: Ableitung
- P: Pumpe
- R: Ringleitung
- W: Wärmeaustauscher

Mittels einer Pumpe P wird neutralisierte Lösung teilweise über die Ringleitung R rückgeführt. Der Rest der neutralisierten Lösung wird über die Ableitung A der weiteren Verwendung zugeführt. Über die Zuleitung Z₁ wird vorzugsweise Natronlauge und über die Zuleitung Z₂ wird vorzugsweise Acrylsäure dosiert.

Damit die Edukte möglichst intensiv in die rückgeführte neutralisierte Lösung eingemischt werden, sollte die Strömung an der Einmischstelle möglichst turbulent sein. Die Einmischstelle ist der Ort, wo das jeweilige Edukt auf die rückgeführte neutralisierte Lösung trifft.

In einer bevorzugten Ausführungsform der vorliegenden Erfindung wird mindestens eines der Edukte in ein, vorzugsweise werden alle Edukte in ein, besonders bevorzugt werden alle Edukte in ein gemeinsames, Venturi-Rohr dosiert.

Ein Venturi-Rohr ist eine in ihrer Länge begrenzte Rohrverengung, in der Druckverlust weitgehend reversibel in kinetische Energie umgewandelt wird. Dazu wird der Querschnitt F₁ auf der Strecke L₁ auf den Querschnitt F₂ vermindert, der Querschnitt F₂ wird auf der Strecke L₂ konstant gehalten und anschließend wird der Querschnitt F₂ auf der Strecke L₃ wieder auf den Querschnitt F₁ geweitet. Dabei ist der Querschnitt F₁ größer als der Querschnitt F₂ und die Länge L₃ größer als die Länge L₁.

Die Dosierung der Edukte für die Neutralisation erfolgt vorzugsweise im Bereich der Strecke L₂ mit dem Querschnitt F₂.

Die optimale Auslegung eines Venturi-Rohrs ist dem Fachmann an sich bekannt. Vorzugsweise wird das Venturi-Rohr so ausgelegt, dass der Druck im Bereich der Strecke L₂ weniger als der Umgebungsdruck beträgt (Saugförderung) und/oder das die Strömung im Bereich der Strecke L₂ turbulent ist, wobei die Reynolds-Zahl mindestens 1000, vorzugsweise mindestens 2000, besonders bevorzugt mindestens 3000, ganz besonders bevorzugt mindestens 4000, und üblicherweise weniger als 10.000 betragen sollte.

Ein weiterer Gegenstand der vorliegenden Erfindung ist ein Verfahren zur Herstellung wasserabsorbierender Polymere, in dem eine gemäß dem erfindungsgemäßen Neutralisationsverfahren hergestellte neutralisierte Lösung als Monomerlösung verwendet wird.

Vorzugsweise wird das erfindungsgemäße kontinuierliche Neutralisationsverfahren mit einem kontinuierlichen Polymerisationsverfahren kombiniert, wobei vorzugsweise alle Verfahrensschritte, wie Neutralisation, Polymerisieren, Trocknen, Mahlen, Sieben, O-berflächennachvernetzen, Sieben, kontinuierlich durchgeführt werden.

Die wasserabsorbierenden Polymerpartikel werden durch Polymerisation einer Monomerlösung hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 70 Gew.-%, besonders bevorzugt von 45 bis 65 Gew.-%, ganz besonders bevorzugt von 50 bis 60 Gew.-%. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung entstehende Polymergel durch beispielsweise gegenläufige Rührwellen kontinuierlich zerkleinert, wie in WO 2001/38402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 50 bis 95 mol-%, besonders bevorzugt von 60 bis 80 mol-%, ganz besonders bevorzugt von 65 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein beheizter Pflugscharmischer verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich zu kleine Polymerpartikel nach der Oberflächennachvernetzung abzutrennen.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzuge Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Obertlächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer, Pflugscharmischer und Schaufelmischer, durchgeführt werden. Besonders bevorzugt sind Horizontalmischer, wie Pflugscharmischer und Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Lödige-Mischer, Bepex-Mischer, Nauta-Mischer, Processall-Mischer und Schugi-Mischer. Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet. Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden. Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Wassergehalt gemäß der von der EDANA (European Disposables and Nonwovens Association) empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA (European Disposables and Nonwovens Association) empfohlenen Testmethode Nr. WSP 242.2-05 "Absorption Under Pressure" bestimmt, wobei statt eines Drucks von 21,0 g/cm² ein Druck von 49,2 g/cm² eingestellt wird.

### Beispiel

In einer kontinuierlichen Apparatur gemäß Figur 1 wurde Acrylsäure mit Natronlauge neutralisiert.

Der Durchmesser der Ringleitung R betrug 20 cm, der Massenstrom in der Ringleitung R vor der Zuführung Z₁ betrug 349 t/h, die Temperatur des Massenstromes in der Ringleitung R vor der Zuführung Z₁ betrug 25 bis 30°C, der Massenstrom Acrylsäure betrug 11,5 t/h, der Massenstrom 50 gew.-%ige Natronlauge betrug 8,4 t/h und der Massenstrom Wasser betrug 15 t/h. Die Temperatur der Eduktmassenströme betrug jeweils ca. 23°C.

Natronlauge und Wasser wurden vorgemischt. Die Natronlauge/Wasser-Mischung und Acrylsäure wurden in ein gemeinsames Venturi-Rohr dosiert. Die Länge des Venturi-Rohres betrug 93,2 cm, wobei sich das Venturi-Rohr über eine Strecke von 8,4 cm auf einen Durchmesser von 10 cm verjüngte, über eine Strecke von 27,6 cm den Durchmesser von 10 cm beibehielt und sich über eine Strecke von 57 cm wieder auf einen Durchmesser von 20 cm weitete. In das Venturi-Rohr mündeten jeweils zwei Zuführungen Z₁ und Z₂. Der Abstand der beiden Zuführungen Z₁ von der Stelle, wo sich das Venturi-Rohr auf 10 cm verjüngt, betrug 5 cm, der Abstand der beiden Zuführungen Z₂ von den beiden Zuführungen Z₁ betrug 8 cm und der Durchmesser der jeweils zwei Zuführungen Z₁ und Z₂ betrug 3,5 cm. Die jeweils beiden Zuführungen Z₁ bzw. Z₂ sind gegenüber angeordnet, wobei die Verbindungsachse der beiden Zuführungen Z₁ gegenüber der Verbindungsachse der beiden Zuführungen Z₂ um 90° gedreht ist.

Der Wärmeaustauscher W war ein Rohrbündelwärmeaustauscher mit einer wirksamen Austauscherfläche von 310 m² und einer Kühlleistung von 2.000 kW, entsprechend einer spezifischen Kühlleistung von 0,645 W/cm². Der Massenstrom der Ringleitung R wurde im Wärmeaustauscher W von 35 bis 40°C auf 25 bis 30°C gekühlt. Die Kühlmitteleingangstemperatur betrug 25 bis 29°C. Der Wärmeaustauscher wurde im Gegenstrom betrieben.

Der hergestellte teilneutralisierte Monomerlösung wies einen Neutralisationsgrad von 65,6 mol-% und einen Feststoffgehalt von 39,6 Gew.-% auf. Die Ringleitung R und die Austragsleitung A waren auch nach längerem Betrieb frei von Ablagerungen.

100g der hergestellten Monomerlösung wurde abgekühlt. Bei einer Temperatur von-7°C schied sich Natriumacrylat an der Gefäßwand ab.

Das Beispiel zeigt, dass sich der Rohrbündelwärmeaustauscher gemäß dem erfindungsgemäßen Verfahren störungsfrei betreiben ließ. Wird dagegen die Monomerlösung weiter abgekühlt, d.h. die spezifische Kühlleistung des Rohrbündelwärmeaustauschers erhöht, so werden Salzablagerungen im Rohrbündelwärmeaustauscher festgestellt, obwohl die Temperatur der abgekühlten Monomerlösung noch deutlich über -7°C beträgt.

## Patentansprüche

1. Verfahren zur Herstellung wasserabsorbierender Polymerpartikel durch Polymerisation einer Monomerlösung, enthaltend
a) mindestens ein ethylenisch ungesättigtes, säuregruppentragendes Monomer,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
wobei das Monomer a) zumindest teilweise neutralisiert wird und die Neutralisationswärme zumindest teilweise mittels eines indirekten Wärmeaustauschers mittels eines Kühlmediums abgeführt wird, **dadurch gekennzeichnet, dass** die spezifische Kühlleistung des Wärmeaustauschers weniger als 5 W/cm² beträgt.

2. Verfahren gemäß Anspruch 1, **dadurch gekennzeichnet, dass** die Temperatur des Kühlmediums im Wärmeaustauscher mindestens 10°C beträgt.

3. Verfahren gemäß einem der Ansprüche 1 oder 2, **dadurch gekennzeichnet, dass** die Monomerlösung im Wärmeaustauscher auf weniger als 40°C gekühlt wird.

4. Verfahren gemäß einem der Ansprüche 1 bis 3, **dadurch gekennzeichnet, dass** der Wärmeaustauscher im Gegenstrom betrieben wird.

5. Verfahren gemäß einem der Ansprüche 1 bis 4, **dadurch gekennzeichnet, dass** der Wassergehalt der Monomerlösung höchstens 70 Gew.-% beträgt.

6. Verfahren gemäß einem der Ansprüche 1 bis 5, **dadurch gekennzeichnet, dass** der Neutralisationsgrad des neutralisierten Monomeren a) mindestens 50 mol-% beträgt.

7. Verfahren gemäß einem der Ansprüche 1 bis 6, **dadurch gekennzeichnet, dass** das Monomer a) Acrylsäure ist.

8. Verfahren gemäß einem der Ansprüche 1 bis 7, **dadurch gekennzeichnet, dass** das neutralisierte Monomer a) zumindest teilweise in die Neutralisation zurückgeführt wird.

9. Verfahren gemäß Anspruch 8, **dadurch gekennzeichnet, dass** zwischen 25 und 95% des neutralisierten Monomeren a) zurückgeführt werden.

10. Verfahren gemäß einem der Ansprüche 1 bis 9, **dadurch gekennzeichnet, dass** 0,05 bis 1,5 Gew.-% Vernetzer b), bezogen auf Monomer a), eingesetzt werden.

## Claims

1. A process for producing water-absorbing polymer particles by polymerizing a monomer solution comprising
a) at least one ethylenically unsaturated monomer bearing acid groups,
b) at least one crosslinker,
c) at least one initiator,
d) optionally one or more ethylenically unsaturated monomers copolymerizable with the monomers specified under a) and
e) optionally one or more water-soluble polymers, by at least partially neutralizing the monomer a) and removing the heat of neutralization at least partially by means of an indirect heat exchanger by means of a cooling medium, wherein the specific cooling performance of the heat exchanger is less than 5 W/cm².

2. The process according to claim 1, wherein the temperature of the cooling medium in the heat exchanger is at least 10°C.

3. The process according to either of claims 1 and 2, wherein the monomer solution is cooled to less than 40°C in the heat exchanger.

4. The process according to any one of claims 1 to 3, wherein the heat exchanger is operated in countercurrent.

5. The process according to any one of claims 1 to 4, wherein the water content of the monomer solution is at most 70% by weight.

6. The process according to any one of claims 1 to 5, wherein the degree of neutralization of the neutralized monomer a) is at least 50 mol%.

7. The process according to any one of claims 1 to 6, wherein the monomer a) is acrylic acid.

8. The process according to any one of claims 1 to 7, wherein the neutralized monomer a) is recycled at least partially into the neutralization.

9. The process according to claim 8, wherein between 25 and 95% of the neutralized monomer a) is recycled.

10. The process according to any one of claims 1 to 9, wherein from 0.05 to 1.5% by weight of crosslinker b), based on monomer a), are used.

## Revendications

1. Procédé de fabrication de particules polymères absorbant l'eau par polymérisation d'une solution de monomères contenant :
a) au moins un monomère éthyléniquement insaturé, portant des groupes acides,
b) au moins un agent de réticulation,
c) au moins un initiateur,
d) éventuellement un ou plusieurs monomères éthyléniquement insaturés copolymérisables avec les monomères indiqués en a) et
e) éventuellement un ou plusieurs polymères solubles dans l'eau,
le monomère a) étant neutralisé au moins en partie et la chaleur de neutralisation étant dissipée au moins en partie par un échangeur de chaleur indirect par un milieu de refroidissement, **caractérisé en ce que** la puissance de refroidissement spécifique de l'échangeur de chaleur est inférieure à 5 W/cm².

2. Procédé selon la revendication 1, **caractérisé en ce que** la température du milieu de refroidissement dans l'échangeur de chaleur est d'au moins 10 °C.

3. Procédé selon l'une quelconque des revendications 1 ou 2, **caractérisé en ce que** la solution de monomères est refroidie à moins de 40 °C dans l'échangeur de chaleur.

4. Procédé selon l'une quelconque des revendications 1 à 3, **caractérisé en ce que** l'échangeur de chaleur est exploité à contre-courant.

5. Procédé selon l'une quelconque des revendications 1 à 4, **caractérisé en ce que** la teneur en eau de la solution de monomères est d'au plus 70 % en poids.

6. Procédé selon l'une quelconque des revendications 1 à 5, **caractérisé en ce que** le degré de neutralisation du monomère a) neutralisé est d'au moins 50 % en moles.

7. Procédé selon l'une quelconque des revendications 1 à 6, **caractérisé en ce que** le monomère a) est l'acide acrylique.

8. Procédé selon l'une quelconque des revendications 1 à 7, **caractérisé en ce que** le monomère a) neutralisé est recyclé au moins en partie dans la neutralisation.

9. Procédé selon la revendication 8, **caractérisé en ce qu'**entre 25 et 95 % du monomère a) neutralisé est recyclé.

10. Procédé selon l'une quelconque des revendications 1 à 9, **caractérisé en ce que** 0,05 à 1,5 % en poids d'agent de réticulation b), par rapport au monomère a), est utilisé.
